# EUROPEAN PATENT APPLICATION

(11) **EP 1 338 295 A1**
(43) Date of publication of application: **27.08.2003**
(21) Application number: 03251117.2
(22) Date of filing: 25.02.2003
(51) Int. Cl.: A61M 5/172, A61M 5/145

(54) **Systems for remotely controlling medication infusion and analyte monitoring**

(30) Priority: 26.02.2002 US 360401 P
(71) Applicant: Lifescan, Inc., Milpitas, California 95035-6312 (US)
(72) Inventor: Bylund, Adam David, Fremont, California 94539 (US); Durban, William Jefferey, Pleasanton, California 94566 (US); Wardle, Michael D., San Jose, California 95138 (US); Long, Karen M., San Jose, California 95125 (US); McClusky, Joseph, Sharon, Massachusetts 02067 (US); Kraft, Ulrich, 65719 Hofheim (DE); Ebner, Manfred, 61440 Oberursel (DE); Steine, Matthias, Inverness IV2 3QG (GB)
(74) Representative: Mercer, Christopher Paul

(57) **Abstract**

Devices, systems and methods are provided for remotely controlling medication delivery to a patient by means of a medication infusion pump, such as a subcutaneous infusion pump, and for remotely controlling the monitoring of one or more physiological fluid analytes such as by a percutaneous measurement device. The systems of the present invention include a medication infusion pump and a hand-held "fob" for the remote control of the infusion pump and/or measurement device. In addition to remotely controlling the insulin pump and the measurement device, the fob provides for the consolidation of blood chemistry data and insulin delivery data over a period of time and maintains such consolidated data for immediate and later retrieval by the user or a physician. The methods of the present invention allow a user to customize and optimize an insulin bolus delivery protocol, *i.e.,* bolus volume and delivery duration, by factoring in or compensating for the user's current or substantially current blood chemistry evaluation and/or the user's anticipated and/or actual carbohydrate intake.

## Description

### Field of the Invention

The invention generally relates to continuous-delivery medication infusion systems and physiological fluid characteristic monitoring systems. More particularly, the invention is related to the user-interactive remote control of such continuous-delivery medication infusion systems and physiological fluid characteristic monitoring systems, as well as the integration of such physiological fluid characteristic monitoring systems within a remote control device.

### Background of the Invention

Medication infusion devices and physiological fluid characteristic monitoring devices are known in the medical field. One very common application of such devices is the delivery of insulin to and the monitoring of blood glucose levels of diabetics. Many advances have been made in recent years, with such device being integrated together to provide an all-in-one device which provides for the controlled delivery of insulin to the patient in accordance with real-time patient blood glucose levels and other requirements.

One such device is disclosed in U.S. Patent No. 5,665,065 which provides for an automatic infusion pump for the continuous, programmed delivery of insulin at a subcutaneous location within the patient. The pump is designed for the programmable delivery of insulin from a reservoir to the patient via tubing implanted within the patient according to a predefined protocol. The pump housing includes an integrated blood sensor for deriving a patient's current blood glucose level. In addition to the current blood chemistry data, the device is configured to receive data from the patient relating to event-specific patient activities, *e.g*., a variation in the patient's exercise or meal schedule or an increase or decrease in the anticipated intake of food, which are likely to affect the patient's current blood chemistry. Such event-specific data and blood characteristics are provided to a central controller/processor housed within the pump-monitor device which modifies the insulin delivery protocol automatically, making the necessary changes in the dosage of insulin and the timing of the delivery of such dosage by the pump.

While such highly automated devices have their advantages, many patients want more direct control over the administration of their medication. For example, a patient may want to stop the administration of medication during a dosage delivery period, even where the initial administration was initiated by the patient rather than according to a preprogrammed algorithm. Circumstances that may present such a situation include, for example, a change in the anticipated intake of carbohydrates by a diabetic, *e.g*., during a meal, a patient finds himself eating an amount of carbohydrates greater or less than what he or she anticipated prior to the meal. Such circumstances may require immediate modification of the then current insulin delivery parameters in effect on the pump.

Accordingly, there is continued interest in the development of new devices and methods for the patient-controlled delivery of medication via a pump which provide even greater flexibility to accommodate the real-time, immediate needs of each patient and to particularly control the real-time delivery of such medication. Of particular interest would be the development of a patient-controlled medication delivery system which provides the patient with such flexibility and control while increasing convenience and ease of use, enhancing portability and providing improved patient privacy when needing to interface with the medication delivery system.

### Summary of the Invention

Devices, systems and methods are provided for remotely controlling medication delivery to a patient by means of a medication infusion pump, such as a subcutaneous infusion pump, and/or for remotely controlling the measurement of physiological fluid, such as blood or interstitial fluid, of a patient by means of a percutaneous physiological fluid monitoring device. The systems of the present invention include a hand-held "fob" for the remote control of the infusion pump and/or the monitoring device. The infusion pump and monitoring device may be separately housed or integrated into a single housing structure.

The infusion pump includes a medication reservoir and a drive motor for dispensing the medication from the reservoir. The infusion pump may further include a power supply and a battery, an alarm, a digital display, a pump controller having a microprocessor for controlling pump operation and pump communication functions, a communication module for the bidirectional communication with the fob and other devices, memory storage means for the short-term or long-term storage of data, and control keys to enter or select data or parameters from menus displayed on the display.

The physiological fluid monitoring device includes a fluid sampling means for accessing and collecting blood or interstitial fluid from the patient and a characteristic measurement means for monitoring one or more characteristics, *e.g*., analytes, of the sampled fluid. The sampling and subsequent monitoring of the physiological fluid may be done on a substantially continuous basis. The physiological fluid monitoring device may further include a power supply and a battery, an alarm, a digital display, a communication module for the bi-directional communication with the fob and other devices, memory storage means for the short-term or long-term storage of data, and user interface control keys to allow the user to enter or select data or parameters from menus displayed on the display. The physiological fluid monitoring device further includes a controller having a microprocessor for controlling operation of the sampling and measurement means, for controlling the receipt and transmission of signals via the communication module and for processing and transferring data between components within the monitoring device. A feature of the physiological fluid monitoring device is that it may be programmed to provide for the continuous, ongoing access, collection and measurement of physiological fluid without the need for human intervention.

The fob includes means for the remotely controlling the pump and/or the continuous physiological fluid collection and monitoring device. Optionally, the fob may also include a "non-continuous" or episodic physiological fluid measurement meter. The fob has a test strip port configured to receive a test strip for the episodic measurement the blood glucose concentration of a sample of the patient's blood by the meter. The fob also contains components which allow a user to remotely control the infusion pump and the physiological fluid collection device, including a fob controller having a microprocessor for controlling pump and meter operation functions and a communication module for communicating pump operation, a display and control keys for the entering, selection and transmission of data to the pump and the physiological fluid collection device, and memory storage means for the storage of such data.

An advantage of the subject system over many conventional insulin delivery and monitoring systems, is the consolidation of an episodic blood chemistry meter and features for the very discrete, remote control of an insulin pump and/or a continuous-measurement analyte tester within a very small, stand-alone fob. In addition to remotely controlling the insulin pump and the continuous measurement analyte tester, the fob provides for the consolidation of blood chemistry data and insulin delivery data over a period of time and maintains such consolidated data for immediate and later retrieval by the user or a physician. As such, a comprehensive analysis can be made of all key information and events affecting the treatment of a patient.

Such advantages are provided by certain features of the subject system which allow a user broad flexibility in the monitoring and in the control of blood glucose levels. Specifically, subject system provides the user with the ability to make changes to bolus and basal rate delivery default parameters at any time. Much of this flexibility is provided by software algorithms for the control and setting of medication boluses.

To better treat a user's immediate and ongoing needs, the present invention allows a user to customize an insulin bolus delivery protocol, *i.e*., bolus volume and delivery duration, by factoring in or compensating for the user's current or substantially current blood chemistry evaluation and/or the user's anticipated and/or actual carbohydrate intake. More specifically, the present invention provides three calculator function options, namely the carbohydrate calculator function, the blood glucose calculator function and the combined calculator function, which allow the user the option to take into consideration either or both blood chemistry and carbohydrate intake, as well as other factors such as exercise undertaken by the user, prior to implementing a bolus delivery protocol. Certain of the parameters for making such calculations are defaults values, *e.g*., the bolus-to-carbohydrate ratio, bolus to blood glucose ratio, and the user's target blood glucose level, which have been preprogrammed into the systems' controllers, while other parameters, *e.g*., the user's actual blood glucose level, the amount of carbohydrates to be consumed, and the bolus dosage correction factors, are to be entered on a real-time basis by the user.

The methods of the present invention involve the remote control of a medication insulin pump and a physiological fluid monitoring device by means of a fob, as described above. Such remote control involves "handshaking" between the pump and the fob and between the monitoring device and the fob (and optionally between the pump and the monitoring device) wherein data and commands are communicated back and forth between the various devices via their respective communication modules.

The methods may involve the implementation of one or more of various types of bolus delivery algorithms which include a standard bolus delivery algorithm, an extended bolus delivery algorithm and a dual bolus delivery algorithm. Each of the above may be implemented with or without the above mentioned calculator functions in order to customize and optimize each bolus delivery protocol at any one given time.

These and other objects, advantages, and features of the invention will become apparent to those persons skilled in the art upon reading the details of the methods and systems of the present invention which are more fully described below.

### Brief Descriptions of the Drawings

Figure 1 illustrates a system of the present invention having a portable medication delivery pump to be worn by the patient and a blood characteristic meter configured in the form a remote control device for controlling the functions of the meter and the pump.
Figure 1A is a view of the pump of Fig. 1 taken along the lines A-A in Fig. 1.
Figure 1B is a view of the remote control-meter device of Fig. 1 taken along the lines BB in Fig. 1.
Figure 2 is a block diagram of the system of Fig. 1.
Figure 3A is a flow chart of the standard bolus delivery algorithm of the present invention.
Figure 3B is a flow chart of the extended bolus delivery algorithm of the present invention.
Figure 3C is a flow chart of the dual bolus delivery algorithm ofthe present invention.
Figure 4A is a flow chart of the carbohydrate calculator mode algorithm of the present invention.
Figure 4B is a flow chart of the blood glucose calculator mode algorithm of the present invention.
Figure 4C is a flow chart of the carbohydrate/blood glucose calculator mode algorithm of the present invention.
Figure 5 is a flow chart of a method of the present invention.
Figure 6 illustrates another system of the present invention having a portable continuous physiological fluid monitoring device to be worn by the patient and a remote control device for controlling the functions of the monitoring device, which remote control device also provides an integral meter for physiological fluid monitoring.
Figure 7 illustrates the continuous physiological fluid monitoring device of the system of Fig. 6 including a disposable cartridge used with the monitoring device.
Figure 8 illustrates an enlarged perspective view of the cartridge of Figure 7.
Figure 9 is a block diagram of the system of Fig. 6.
Figure 10 is a block diagram of another system of the present invention which includes a portable medication delivery pump, a continuous physiological monitoring device and a remote control for controlling the functions of the delivery pump and the monitoring device.

### Detailed Description of the Invention

Before the present invention is described, it is to be understood that this invention is not limited to the particular embodiments described, as such may, of course, vary. It is also to be understood that the terminology used herein is for the purpose of describing particular embodiments only, and is not intended to be limiting, since the scope of the present invention will be limited only by the appended claims.

Where a range of values is provided, it is understood that each intervening value, to the tenth of the unit of the lower limit unless the context clearly dictates otherwise, between the upper and lower limit of that range and any other stated or intervening value in that stated range is encompassed within the invention. The upper and lower limits of these smaller ranges may independently be included in the smaller ranges is also encompassed within the invention, subject to any specifically excluded limit in the stated range. Where the stated range includes one or both of the limits, ranges excluding either or both of those included limits are also included in the invention.

Unless defined otherwise, all technical and scientific terms used herein have the same meaning as commonly understood by one of ordinary skill in the art to which this invention belongs. Although any methods and materials similar or equivalent to those described herein can also be used in the practice or testing of the present invention, the preferred methods and materials are now described.

It must be noted that as used herein and in the appended claims, the singular forms "a", "an", and "the" include plural referents unless the context clearly dictates otherwise. Thus, for example, reference to "a test strip" includes a plurality of such test strips and reference to "the device" includes reference to one or more devices and equivalents thereof known to those skilled in the art, and so forth.

The publications discussed herein are provided solely for their disclosure prior to the filing date of the present application. Nothing herein is to be construed as an admission that the present invention is not entitled to antedate such publication by virtue of prior invention. Further, the dates of publication provided might be different from the actual publication dates which may need to be independently confirmed.

The present invention will now be described in detail. In further describing the present invention, the subject systems and device components will be described first. Next, various methods of using the subject devices and systems as well as methods for controlling the testing of physiological sample characteristics and for controlling the delivery of medication to a patient will then be described. Finally, a brief description is provided of the subject kits, which kits include the subject devices and systems for use in practicing the subject methods.

In the following description, the present invention will be described in the context of glucose concentration measurement and insulin delivery applications; however, such is not intended to be limiting and those skilled in the art will appreciate that the subject devices, systems and methods are useful in the measurement of other physical and chemical characteristics, *e.g.,* blood coagulation time, blood cholesterol level, etc., of biological substances and in the delivery of other medications and the like, *e.g*., pain control medication, antibiotics, chemotherapy and nutritional therapy.

### Systems and Devices

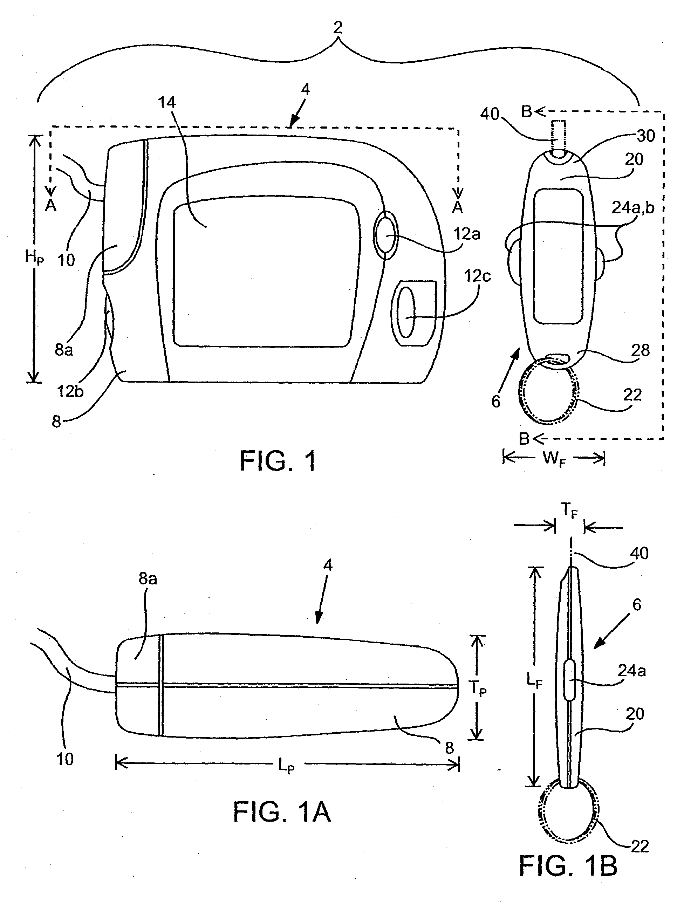

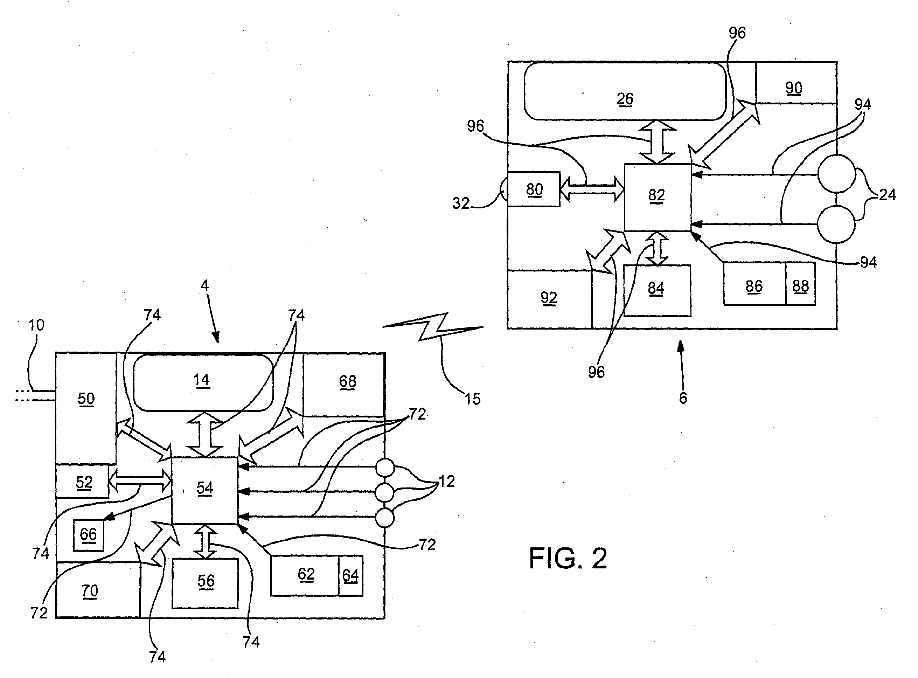

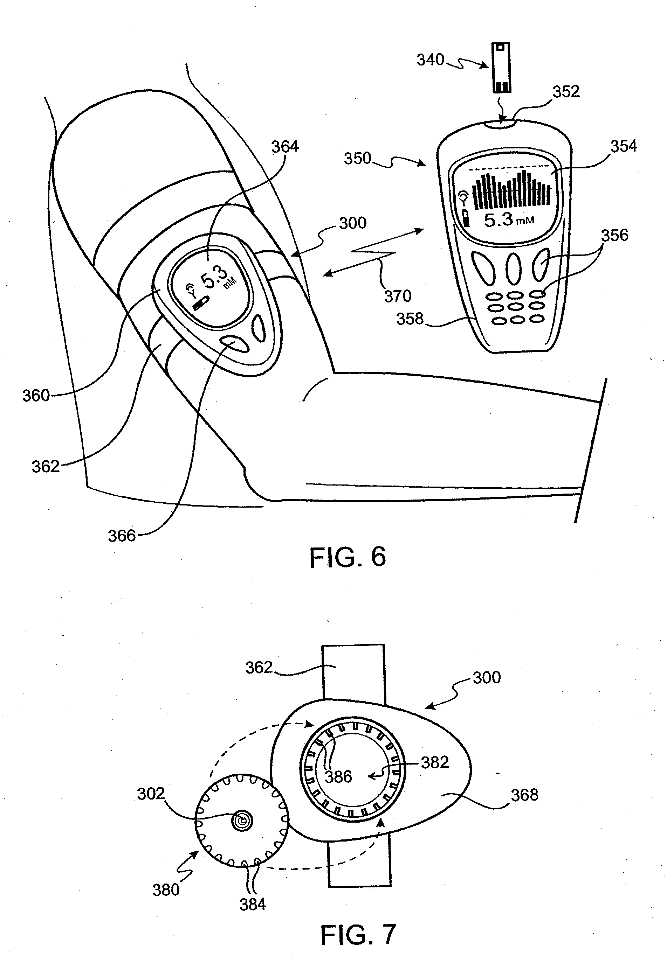

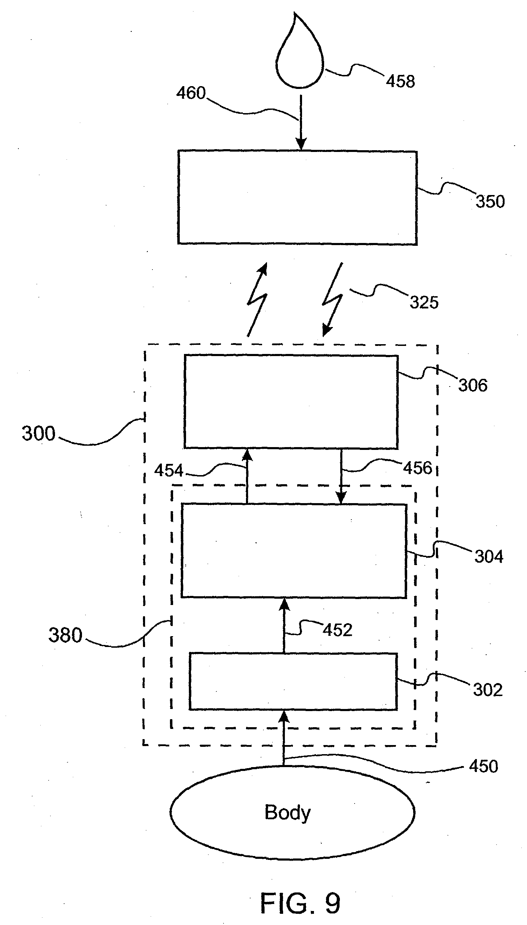

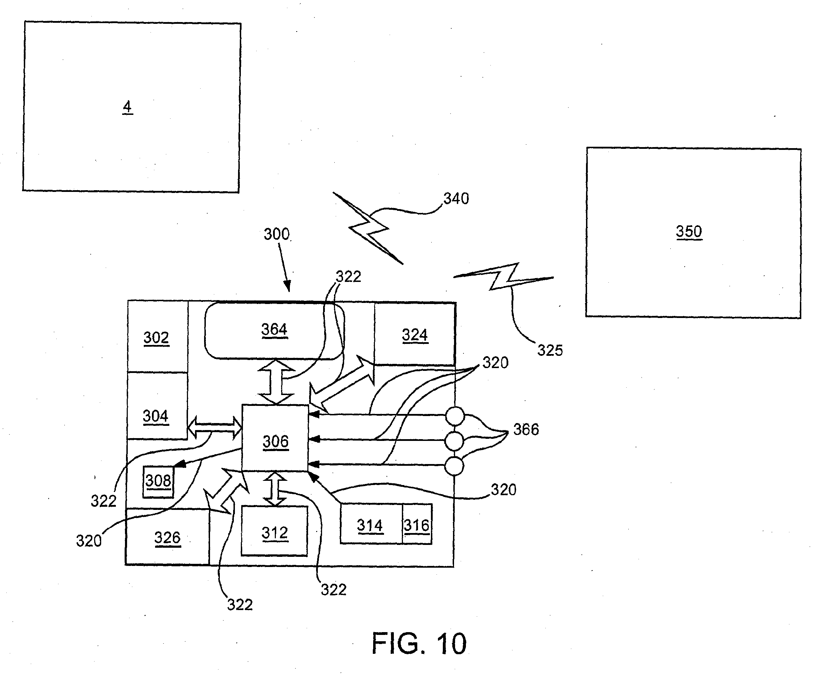

Referring now to the drawings, Figs. 1 and 2 illustrate a system of the present invention having an infusion pump 4 and a remote control device 6, commonly referred to as a "fob." Fig. 1A shows a top view of pump 4 along the line A-A and Fig. 1B shows a side view of device 6 along the line B-B. Fig. 2 illustrates block diagrams of pump 4 and fob 6 and their respective components. Figs. 6-9 illustrate another system of the present invention having a physiological fluid monitoring device 300 and a fob 350. Figs. 6-8 illustrate external views of the components of the system and Fig. 9, along with Fig. 10, provide block diagrams of the monitoring device 300 and fob 350. Fig. 10 illustrates another system of the present invention which includes both a pump and a monitoring device.

### Infusion Pump

Infusion pump 4 has a housing 8, preferably formed from a durable plastic material, having a portion 8a adapted to receive or house a syringe or reservoir (not shown) holding prescribed medication for administration to the patient via an associated indwelling infusion tubing or catheter 10. Housing 8 is preferably sufficiently compact so as to be comfortably and discretely carried by the user, for example, by means of a belt clip or the like. Generally, housing 8 has a length L_{P} in the range from about 2.5 to about 5 inches and more typically from about 3 to about 3.5 inches, a height H_{P} in the range from about 1.5 to about 3 inches and more typically from about 2 to about 2.5 inches, and a thickness Tp in the range from about 0.5 to about 1.5 inches and more typically from about 0.75 to about 1 inch. While pump 4 is illustrated having a substantially rectangular or square shape, it may have any appropriate shape, for example, circular, oblong, etc.

Infusion pump 4 houses many of the same basic components and construction as prior art infusion pumps, such as those disclosed in U. S. Patent Nos. 4,562,751, 4,678,903, 5,080,653, 5,097,122, 5,935,099, 6,248,093 B1 and 6,406,605 B1 which are herein incorporated by reference. Such basic components include a medication reservoir 50 and a drive motor 52 which uses a lead screw assembly for motor-driven advancement of a reservoir piston (not shown) to cause the medication to exit from a pump outlet into infusion tube 10; however, other suitable mechanisms for dispensing medication from reservoir 50 may be used such as, for example, electroosmotic flow (also referred to as electrokinetic flow). Additionally, a power supply 62 and a battery 64 are provided to supply the necessary electrical power for operating the components of pump 4.

Examples of electroosmotic pumps suitable for pumping a medication are disclosed in U.S. Patent Nos. 6,406,605, 3,923,426 and PCT publication WO 02/094440. Basically an electro-osmotic pump comprises a pump medium to be wetted by the liquid to be pumped and a pair of electrodes to impose a voltage over the pump medium in the direction of flow. Often the pump medium is in the form of a porous membrane exhibiting a net electrical surface charge when wetted by the liquid to be pumped. The electric field set up between the electrodes results in a shifting of charged species in the liquid in the direct vicinity of the surface of the pump medium. This transport of charged species drags along the liquid to be pumped and results in the required liquid flow in direction of the electric field. Each of the disclosed pumps can be applied to either directly or indirectly pump the medication. In direct pumping, the medication flows though the pump medium whilst in indirect pumping a second liquid is pumped through the pump medium and the displacement of this second liquid is applied to pressurize and drive the medication to be delivered.

Pump 4 may further include audio, visual and/or vibration alarm/reminder means 66 for alerting the user to an alarm condition, *e.g*., when a low volume of medication is remaining in the reservoir, a blood chemistry measurement which is outside the acceptable range, low battery power, when an occlusion occurs in the infusion tubing, when there is a malfunction in the pump, or for reminding the user of an event or to perform a necessary action, *e.g*., perform a blood chemistry evaluation, enter medication delivery protocol, etc. or some other user-definable alert. Suitable alarm/reminder means 66 for use with pump 4 may include audio means, *e.g*., a piezoelectric beeper; motion means, *e.g*., a vibration motor; and/or visual means, *e.g.,* an LED, etc.

Pump 4 also includes a display 14, such as a liquid crystal display (LCD), for graphic and alphanumeric display. Such graphic display may include icons representative of, for example, bolus and basal rate delivery status and settings, historical data regarding blood glucose levels and insulin deliveries stored in memory, stop bolus commands, etc. Selecting an icon will bring up the corresponding user interface menu.

Pump 4 further includes a pump controller 54 having a microprocessor for controlling pump operation and pump communication functions. Pump controller 54 may also have a memory element for storing pump operation software programs and other static data such as pre-programmed default values including but not limited to blood chemistry meter calibration information, user preferences, *e.g*., language, user basal rate, carbohydrate and blood glucose bolus correction factors, a user's target blood glucose level, calculator, etc.

A memory storage means 56 is provided for the temporary storage of dynamic data such as pump infusion data, blood chemistry data (acquired by meter/sensor 80 of fob 6) and other data entered by the user. Pump infusion data may include information such as the medication delivery rate (Units/Hour), the current volume of medication held in the reservoir, bolus delivery start/stop time, bolus delivery duration, etc. Blood chemistry data includes the blood glucose concentration (mg/dL) measurements and their respective dates and times. Other data that may be entered by the user via control keys 12 include but are not limited to carbohydrate intake (mmol/L) and the parameters related to bolus deliveries, *e.g*., bolus dosage, bolus duration, bolus start and stop times, etc.

Pump 4 further includes control keys 12a, 12b and 12c to allow the user to enter or select data or parameters from a menu displayed on display 14. For example, control key 12a may have a jogwheel configuration, as illustrated in Fig. 1A. More specifically, jogwheel 12a is rotated by the user to select the desired volume of the medication bolus to be delivered by pump 4. Jogwheel 12a may also be used to scroll through menu items from display 14 and to select such menu items by depressing jogwheel 12a Control keys 12b and 12c may be configured as depressible buttons for initiating the communication of data to and from fob 6 or other auxiliary devices and for initiating a bolus delivery. Pump 4 may have any number of control keys, each having any suitable configuration, *e.g.,* jog wheel, depressible button, keypad, etc., for controlling pump 4.

Commands and data are communicated to and from pump controller 54 via one-way and two-way data lines or buses 72 and 74, respectively. More specifically, pump controller 54 receives electrical power from power supply 62 and battery 64, receives input data and commands from the user via control keys 12, and transmits commands to alarm/reminder means 66 on one-way lines 72; otherwise, communication between pump controller 54 to and from the various components of pump 4 is accomplished by two-way lines 74. The communication of information between pump 4 and fob 6 and other external devices is described in greater detail below.

### Pump Remote Control / Blood Chemistry Meter ("Fob")

Fob 6 includes an episodic blood characteristic measurement sensor or meter and means for the remote control of pump 4. Fob 6 has a housing 20, preferably formed from a durable plastic material and having a very compact size and an ergonomic shape so as to be discretely carried in one's clothing, such as a pocket, or held in one's hand. Generally, fob 6 has a size no greater than about one-third the size of pump 4. Fob housing 20 has a length L_{F} in the range from about 1.5 to about 4 inches and more typically from about 2 to about 2.5 inches, a width W_{F} in the range from about 0.75 to about 2 inches and more typically from about 1 to about 1.5 inches, and a thickness T_{F} in the range from about 0.25 to about 1 inch and more typically from about 0.5 to about 0.75. While fob 6 is illustrated as having a substantially oblong or elliptical shape, it may have any shape, *e.g*., circular, etc., preferably an ergonomic shape. Fob 6 may be further configured, such as at its proximal end 28, to provide attachment to an accessory ring 22, which may be used to secure fob 6 to an item of clothing or to keys and the like.

At the fob's distal end 30 is a test strip port 32 configured to receive a test strip 40, such as an electrochemical, colorimetric or photometric test strip used in analyte concentration determination, such as the glucose concentration in a sample of blood taken from a user. Housed within fob 6 is a meter 80 for making such determinations. Test strip port 32 and meter 80 may also be configured to receive a calibration strip or the like for calibrating meter 80.

Examples of electrochemical test strips suitable for use with the subject invention include those described in copending U.S. Application Serial Nos. 09/497,269; 09/736,788 and 09/746,116, U.S. Patent Nos. 6,475,372; 6,193,873; 5,708,247; 5,951,836; 6,241,862; 6,284,125; and 6,444,115, and International Patent Application Publications WO/0167099; WO/0173124; WO/0173109; and WO/0206806, the disclosures of which are herein incorporated by reference. Examples of colorimetric or photometric test strips suitable for use with the subject invention include those described in U.S. Patent Nos. 5,563,042; 5,753,452; and 5,789,255, herein incorporated by reference. Certain aspects of the functionality of electrochemical meters suitable for use with the subject systems are disclosed in U.S. Patent No. 6,193,873, as well as in copending, commonly owned U.S. Application Serial Nos. 09/497,304, 09/497,269, 09/736,788, 09/746,116 and 09/923,093, the disclosures of which are herein incorporated by reference. Certain aspects of the functionality of colorimetric/photometric meters suitable for with the present invention use are described in, for example, U.S. Patent Nos. 4,734,360, 4,900,666, 4,935,346, 5,059,394, 5,304,468, 5,306,623, 5,418,142, 5,426,032, 5,515,170, 5,526,120, 5,563,042, 5,620,863, 5,753,429, 5,773,452, 5,780,304, 5,789,255, 5,843,691, 5,846,486, 5,968,836 and 5,972,294, the disclosures of which are herein incorporated by reference.

In addition to housing test strip meter 80, fob 6 contains components which allow a user to remotely control infusion pump 4. Such components include a fob controller 82 which, similar to pump controller 54, includes a microprocessor for controlling pump and sensor/meter operation functions and for communicating pump operation functions and blood chemistry information to pump 4 or to another external device from fob 6. Fob controller 82 may also have a memory element for storing pump and sensor operation software programs.

Fob 6 further includes a memory storage means 84 for the storage of dynamic data such as blood chemistry data and other data entered by the user. Memory storage means 84 stores a limited number, about 10, more or less, of the latest blood chemistry measurements and corresponding dates and times of such measurements, event-specific user parameters, *e.g.,* exercise duration, carbohydrate intake, etc.

Control keys 24, which may have a jogwheel and/or depressible button configurations similar to control keys 12 of pump 4, allow a user to enter or select data from program menus displayed on a display 26, such as a liquid crystal display (LCD), for displaying graphic and alphanumeric information. Typically, a jogwheel is used to select or retrieve data or to select a bolus delivery program or parameters to be implemented. A depressible button is most often used to transmit data, *e.g.,* glucose results, bolus delivery commands, silence alarm commands, terminate bolus delivery commands, etc., to pump 4 or to another external device. The data entered or selected by the user via control keys 24 is sent to controller 82 for implementing a sensor or pump function or otherwise storing such data in memory storage means 84. Fob 6 may have any number of control keys, each having any suitable configuration, *e.g.,* jogwheel, depressible button, key pad, etc.

Data that may be entered by the user via control keys 24 includes, but is not limited to, carbohydrate intake (grams), the desired bolus delivery program and the parameters related to bolus deliveries, *e.g*., bolus dosage, bolus duration, bolus start and stop times, type of medication, amount of medication, target gluocse range (both upper and lower), exercise intensity, exercise duration, health comments, food type, food amount, HbAlc, blood pressure, and other data as disclosed in Great Britain Patent No. GB0212920.3, etc., as well as the provision of a user-operated calculator for determining and setting the appropriate bolus volume.

Commands and data are communicated to and from fob controller 82 via one-way and two-way data lines or buses 94 and 96, respectively. More specifically, fob controller 82 receives electrical power from power supply 86 and battery 88 and receives input data and commands from the user via control keys 24 on one-way lines 94; otherwise, communication between fob controller 82 to and from the various components of fob 6 is accomplished by two-way lines 96. The communication of information between fob 6 and pump 4 and other external devices is described in greater detail below.

### Continuous Physiological Fluid Monitoring Device

Fig. 6 illustrates another system of the present invention including a continuous physiological fluid monitoring device 300 and a remote control device 350. Remote control device or fob 350 may be similar to the structure and function of fob 6 as described above, and may optionally include a physiological fluid measurement meter for the non-continuous or episodic analyte testing of physiological fluid. As such, fob 350 is provided with a test strip port 352 for receiving a test strip 40, as described above. As with fob 6, fob 350 has a low-profile housing 358, a display 354, control keys 356 and the same or similar internal componentry (not shown).

Monitoring device 300 also has a low profile housing 360 and a strap 362 which allows it to be worn on a limbic region such as the arm. In Fig. 6, device 300 is shown worn around a patient's upper arm but may also be configured to be worn around the forearm or wrist. Monitoring device 300 is also provided with a display 364 and control keys 366 similar to the displays and control keys described above. As shown in Fig. 7, the underside or skin-contacting side 368 of housing 360 is configured with an insertion cavity 382 to receive a disk-shaped, disposable cartridge 380 which includes a fluid sampling means 302 operatively connected and in fluid communication with a measurement sensor or means 304 housed within the cartridge. The measurement sensor 304 may have an electrochemical or photometric/colorimetric configuration and have the ability to measure glucose semi-continuously or continuously similarly to those meters disclosed in WO 02/49507A1, which is incorporated herein in its entirety. In the embodiment of Fig. 7, the measurement sensor has an electrochemical configuration with electrical communication established between the sensor and the electronics of monitoring device 300 by means of a set of electrical contact pad pairs 384 on the circumference of cartridge 380 and corresponding electrical contact pins 386 within insertion cavity 382.

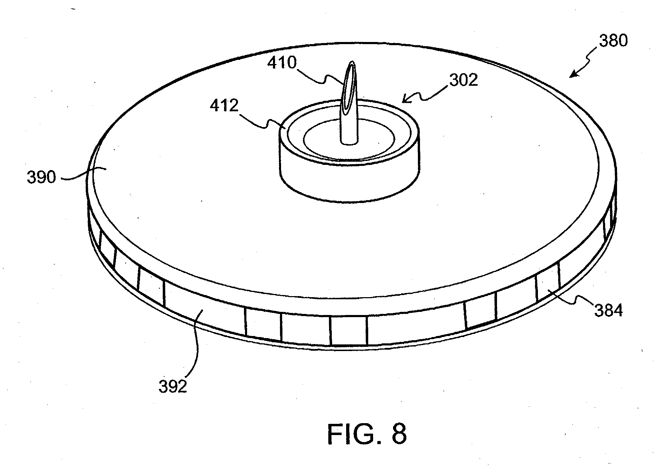

Fig. 8 illustrates an enlarged perspective view of cartridge 380. Cartridge 380 is formed of a molded base 392 having a disk shape and having a diameter in the range from about 20 mm to about 40 mm, and more typically about 35 mm, and a thickness in the range from about 0.1 mm to about 3 mm, and more typically about 2 mm.

A sampling means 302 in the form of a needle 410 and a pressurizing ring 412 are provided on the bottom surface 390 of base 302. Needle 410 is used to penetrate the skin of the user and for accessing and extracting physiological fluid. Needle 410 has an inner diameter in the range from about 0.1 mm to about 0.5 mm is most typically about 0.3 mm (25 gauge). Pressurizing ring 412 functions to stabilize and pressurize the area of skin surrounding the penetration site in order to actively facilitate the extraction of ISF into needle 410. To accomplish these functions, pressurizing ring 412 typically has a diameter in the range from about 5 mm to about 30 mm, and more typically has a diameter of about 12 mm. Needle 410 is housed is positioned at its proximal end within a recess (not shown) of pressurizing ring 412. Needle 302 preferably has a penetration length dimensions which allows it to penetrate the skin to a depth which minimizes the pain felt by the user. The depth of the recess determines the maximum penetration depth of needle 410. As such, 410 needle may be configured and positioned relative to pressurizing ring 412 to penetrate only into but through the dermis layer of skin where there is substantially blood free interstitial fluid (ISF), typically to a depth from about 1.5 mm to about 3.0 mm below the skin surface.

Preferably, needle 410 and pressurizing ring 412 move and are applied to the skin independently of each other. In practice, a driving means, such as a spring, is used to urge pressure ring 412 against the skin, and a second driving means, such as a second spring, is used to launch needle 410 into the skin. While such mechanisms are not specifically illustrated or described herein, such mechanisms are known by those skilled in the art.

Needle 410 is in fluid communication with at least one or more sensors or detectors housed within cartridge 380 to carry out the analyte measurement function of device 300. Suitable cartridges or the like for use with monitoring device 300 are disclosed in commonly owned and assigned International Publication WO 02/49507, which is incorporated herein in its entirety.

Fig. 9 provides a schematic illustration of the function of system of Fig. 6. As shown in Fig. 9, the system generally includes monitoring device 300 and remote control fob 350. Monitoring device 300 includes a disposable cartridge 380 electronically interfaced with a controller 306. Cartridge 380, as mentioned above, includes a sampling means 302 in fluid communication with a sensor means 304. Sampling means 302 has fluid access means, such as a needle, external to the device housing for extracting physiological fluid, *e.g*., ISF, from the body. In operation, fluid accessed in the skin is transferred 450 into the fluid collection areas of the sampling means 302. The sampled fluid is then transferred 452 into the sensor means 304 where the selected analyte is measured. Signals representative of the measurement values are input 454 to controller 306 which controls fluid sample measurement operation via output signals 456 (see Fig. 10). Representations of those values are then displayed on display 364 for observation by the user. This data is then also communicated to remote control fob 350 via bi-directional communication signals 325, described in greater detail below. As mentioned above, fob 350 may also be provided with a sensor mechanism for measuring analyte concentration from sampled fluid 458, typically blood, applied to a test strip and inserted 460 into fob 350 for testing by the sensor mechanism. The remote sensor of fob 350 may be used for calibrating the local sensors of monitoring device 300.

Fig. 10 further illustrates the internal componentry of monitoring device 300. As mentioned above, controller 306 has a microprocessor for controlling fluid sample measurement operation and communication between components of device 300 and for controlling communication between monitoring device 300 and fob 350 via bi-directional communication protocol 325. Controller 306 may also have a memory element for storing sensor operation software programs and other static data such as pre-programmed default values including but not limited to sensor calibration information, alarms, and unit identification/serial number.

Measurement device 300 may further include audio, visual and/or vibration alarm/reminder means 308 for alerting the user to an alarm condition, *e.g*., when blood glucose levels falls outside the acceptable range, when battery power is low, when the fluid sample or sensor is malfunctioning, or for reminding the user of an event or to perform a necessary action, *e.g.,* replacing the sampling means. Suitable alarm/reminder means 308 may include audio means, *e*.*g*., a piezoelectric beeper; motion means, *e.g.,* a vibration motor; and/or visual means, *e.g*., an LED, etc.

Measurement device 300 also includes a display 364, such as a liquid crystal display (LCD), for graphic and alphanumeric display of data such as sample fluid test results, *e.g.,* blood glucose levels, and calibration results. A memory storage means 312 is provided for the temporary storage of dynamic data such as blood chemistry data acquired by sensor means 304 and data entered by the user. Blood chemistry data includes the blood glucose concentration (mg/dL) measurements and their respective dates and times. Other types of data storable on memory storage means 312 includes but are not limited to the type of medication, amount of medication, target gluocse range (both upper and lower), exercise intensity, exercise duration, health comments, food type, food amount, HbA1c, blood pressure, etc. Additionally, a power supply 314 and a battery 316 are provided to supply the necessary electrical power for operating the components of measurement device 300.

Measurement device 300 further includes control keys 366 to allow the user to enter or select data or parameters from a menu displayed on display 364, such as inputs for turning the device on and off, temporarily suspending operation, turning off RF transmission (in restricted areas), alarm acknowledgement and reset, and synchronizing communications with other devices. As with the control keys of pump 4 and fob 6, control keys 366 may have any number of control keys, each having any suitable configuration, *e.g.*, jog wheel depressible button, keypad, etc., for controlling measurement device 300.

Commands and data are communicated to and from controller 306 via one-way and two-way data lines or buses 320 and 322, respectively. More specifically, controller 306 receives electrical power from power supply 314 and battery 316, receives input data and commands from the user via control keys 318, and transmits commands to alarm/reminder means 308 on one-way lines 320; otherwise, communication between controller 306 to and from the various components of measurement device 300 is accomplished by two-way lines 322. The communication of information between measurement device 300 and fob 350 and other external devices is described in greater detail below.

The systems of the present invention may include both an infusion pump 4 as well a continuous physiological fluid monitoring device 300, as illustrated in Fig. 10, where bi-directional communication 340 between pump 4 and device 300 is also provided. While infusion pump 4 and measurement device 300 have been described as separate components, the medication delivery and physiological sampling and measurement components and functions may be combined into an integrated device whereby they share power, controller, alarm, display, memory and communication components. Such an integrated unit provides the advantage of requiring the patient to carry or wear only one piece of hardware rather than two.

### Communication and Data Transmission

The bidirectional communication (designated by reference number 15 in Fig. 2, reference number 325 in Figs. 9 and 10 and reference number 340 in Fig. 10) and transfer of data between pump 4 and fob 6 and between measurement device 300 and fob 350 may be accomplished by any suitable means, *e.g*. radio frequency (RF) transmission, infrared (IR) transmission, etc. For example, pump 4 and fob 6 may each have an RF communication module 68 and 92, respectively, which are controlled by their respective controllers, allowing bidirectional communication between the two devices provided the devices are within a maximum range of each other. Typically, such range is within about 0 to about 10 ft and more typically within about 0 to about 4 ft, and usually no more than about 25 feet. Similarly, measurement device 300 may have an RF communication module 324 controlled by controller 306 which provides for bi-directional communication between the measurement device 300 and fob 350. Additionally, pump 4 and measurement device 300 may communicate directly with each other in the same bi-directional manner or through a fob. In systems where the infusion pump and measurement devices are integrated into a single unit, communication is handled by a common controller or microprocessor.

Modules 68, 92 and 324 are configured and programmed to "link" a particular pump unit and/or measurement device to a particular fob unit to prevent unintentional communications between the fob and other infusion pumps and measurement devices within the same frequency range. The communication protocol between a pump-fob or measurement device-fob or pump/measurement device-fob combination may be configured so as to provide an address associated with the pair which precedes every data transmission between the two so as to prevent inadvertent transmissions between one user's system and another user's system. The modules may be further configured to link a fob to more than one pump and/or measurement device belonging to the same user, as some users have more than one pump. Likewise, the modules may be programmed to link one pump and/or measurement device to more than one fob belonging to the same user.

While the majority of data and information transferred between pump 4 and fob 6 is initiated by the user, there are certain communications between the two devices which are automatic and do not require user intervention. The modules may be configured, for example, such that blood chemistry data and user preference information, *e.g*., language, bolus limits, etc., is periodically sent from the fob to the pump or from the pump to the fob, or is automatically sent upon turning on the fob. Also, the modules may be configured such that medication infusion information, *e.g*., historical basal rate and bolus delivery data, is periodically sent from the pump to the fob.

Similarly, information regarding the patient's blood glucose levels, as monitored by measurement device 300, may be automatically communicated on either a continuous or periodic basis to fob 350. Particularly in those embodiments of measurement device 300 where an alarm means is not provided, fob 6 may be provided with an alarm and alert the patient when the patient's blood glucose level falls outside an acceptable range which may be defined by a user or a doctor. The system may be programmed to automatically adjust the then in process insulin delivery protocol or prompt the patient to override the protocol via inputs to fob 350.

The system may be further configured such that pump 4 and measurement device 300 are able to communicate with each other. Where pump 4 and measurement device 300 are separate components, they may communicate by means of bi-directional communication 340 similar to the manner in which pump and fob 6 communicate with each other. In an integrated unit, the functions of medication delivery pump and the fluid sampling and measurement means are controlled by a common controller (not shown). In either case, the system may be programmed such that medication protocol implemented by pump 4 is automatically adjusted based on the analyte measurement levels determined by measurement device 300 via commands to and from fob 6. Such may be accomplished without any intervention by the patient and even without the patient being notified or otherwise aware of the adjustment in protocol.

Each of pump 4, fob 6 or 350 and measurement device 300 may optionally include communication modules and/or input/output ports 70, 90 and 326, respectively, (such as an RS232 (IEEE standard) or a Universal Serial Bus (USB)) for communicating with external devices such as personal computers (PC), personal digital assistants (PDAs) and the like. In an embodiment of this invention, the communication modules may use a wireless communication method such as a Bluetooth or a Wi-Fi 802.11 scheme. For example, the data stored in either or each of the pump, the fob or the measurement device's controller and memory storage means may be downloaded to an external computer for detailed review and analysis or further processing by a physician to determine, for example, the effectiveness of the drug regime, patient compliance or trends in the patient's glucose levels. Conversely, the physician may use an external computer to download software programs and operational parameters, *e.g.*, the patient's basal rate and certain customized target values, ranges, reminders and alarms, to the pump and/or fob controllers. Communication between the devices of the present invention and external devices may be provided by telemetry transmission, *e.g.*, RF, IR, etc., or data port technologies, *e.g.*, modem, cable, etc.

In an embodiment of the invention, fob 6 also incorporates a portion of storage means 84 that will allow future updates ("field upgrade") of the operating system and or other software elements. Preferably, a portion of storage means 84 is of the type "flash memory" which does not need a electrical energy in order to securely store its contents.

Fob 6 may further comprise a communication slot (not shown) for receiving a data-carrying element and communicating therewith. This data-carrying element preferably is a 'SIM' card type device. A single use data-carrying element is provided with or on every disposable cartridge, and contains production lot specific data (calibration data, identification number etc.). The data-carrying element is read-out by the remote controller and the data received therefrom is applied in the interpretation of the ISF glucose data received from fob 6.

### Software Algorithms

Each of the components of the subject systems is provided with software which enables the components to perform their various functions and to communicate with each other. Certain features and algorithms of the software used with the present invention is provided in detail below.

An advantage of the subject system over many conventional insulin delivery systems, is the consolidation of a blood chemistry meter and features for the remote control of an insulin pump within a very small, stand-alone device such as the fob just described. In addition to remotely controlling the insulin pump and the measurement device, the fob provides for the consolidation of blood chemistry data and insulin delivery data over a period of time and maintains such consolidated data for immediate and later retrieval by the user or a physician. As such, a comprehensive analysis can be made of all key information and events affecting the treatment of a patient.

Such advantages are provided by certain features of the subject system which allow a user broad flexibility in monitoring and in the control of blood glucose levels. Specifically, subject system provides the user with the ability to make changes to bolus and basal rate delivery default parameters at any time. Much of this flexibility is provided by software algorithms for the control and setting of medication boluses.

Controller 54 of pump 4 and controller 82 of fob 6 are programmed with software that supports several types of bolus delivery protocols: standard, extended and dual. The standard or "quick" bolus delivery protocol allows the user to select a dosage of medication for immediate infusion of the entire bolus. The user is likely to require such a quick bolus delivery immediately prior to a meal or snack that includes simple carbohydrates, *e.g.*, fruits, etc. The extended bolus delivery protocol allows the user to select a dosage of medication for infusion over a selected period of time within in a certain time range. An extended bolus delivery protocol is typically implemented prior to a meal that includes a sizable portion of complex carbohydrates, *e.g*., starches, etc. The dual bolus delivery protocol combines the above two protocols, allowing a user to consecutively implement both a quick bolus and an extended bolus in a single command sequence. A dual bolus delivery protocol is typically implemented prior to eating a meal containing both simple and complex carbohydrates.

To better treat a user's immediate and ongoing needs, the present invention allows a user to customize an insulin bolus delivery protocol by factoring in or compensating for the user's current or substantially current blood chemistry evaluation and/or the user's anticipated and/or actual carbohydrate intake. More specifically, the present invention provides three calculator function options, namely the carbohydrate calculator function, the blood glucose calculator function and the combined calculator function, which allow the user the option to take into consideration either or both blood chemistry and carbohydrate intake, as well as more minor factors such as exercise undertaken by the user, prior to implementing a bolus delivery protocol. While all three bolus delivery algorithms may provide for all three calculator function options, typically it is not appropriate to factor in blood chemistry data for extended bolus deliveries (either alone or in combination with a quick bolus delivery) as blood chemistry is likely to change after a relatively short period of time, *i.e*., prior to the completion of an extended bolus delivery.

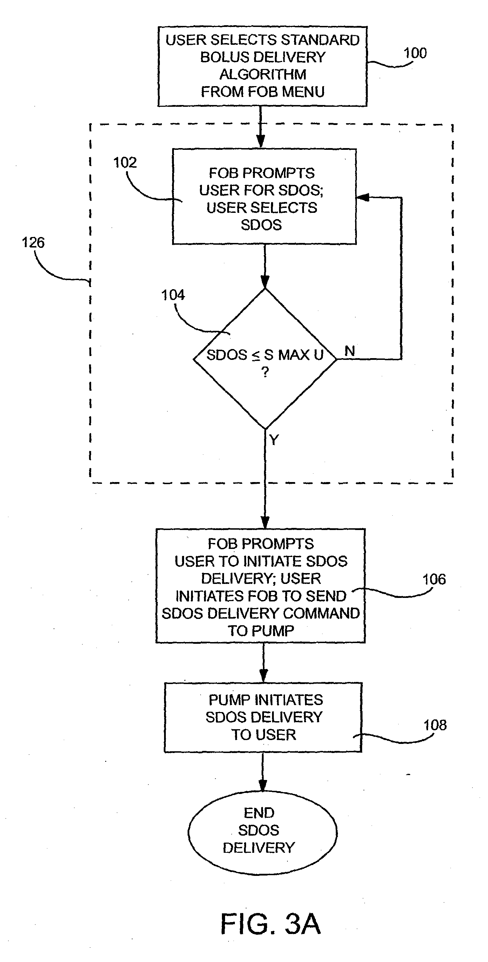

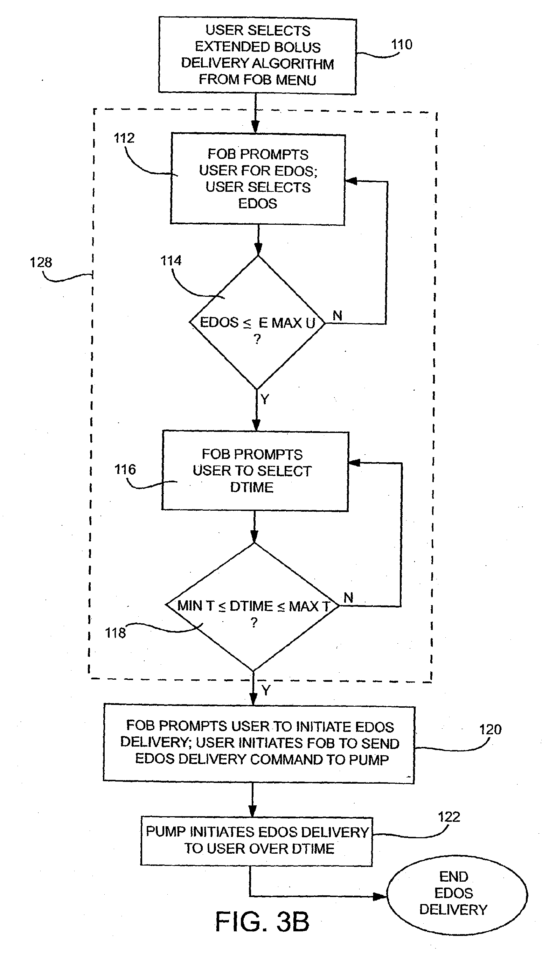

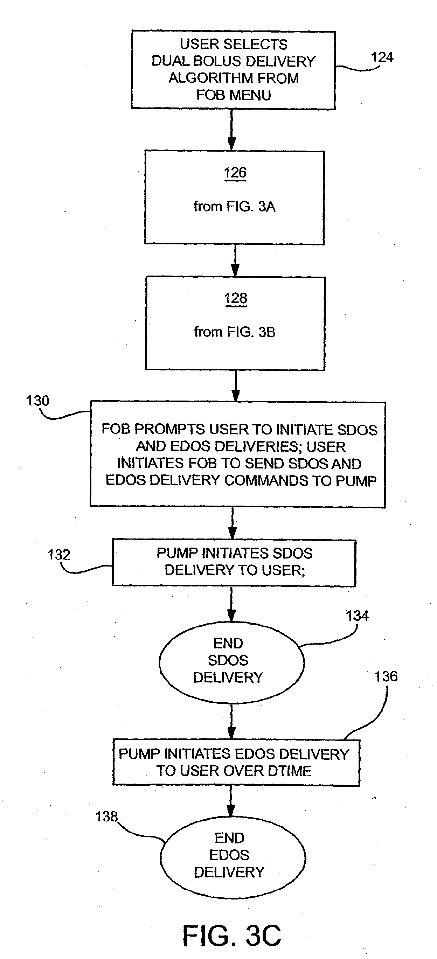

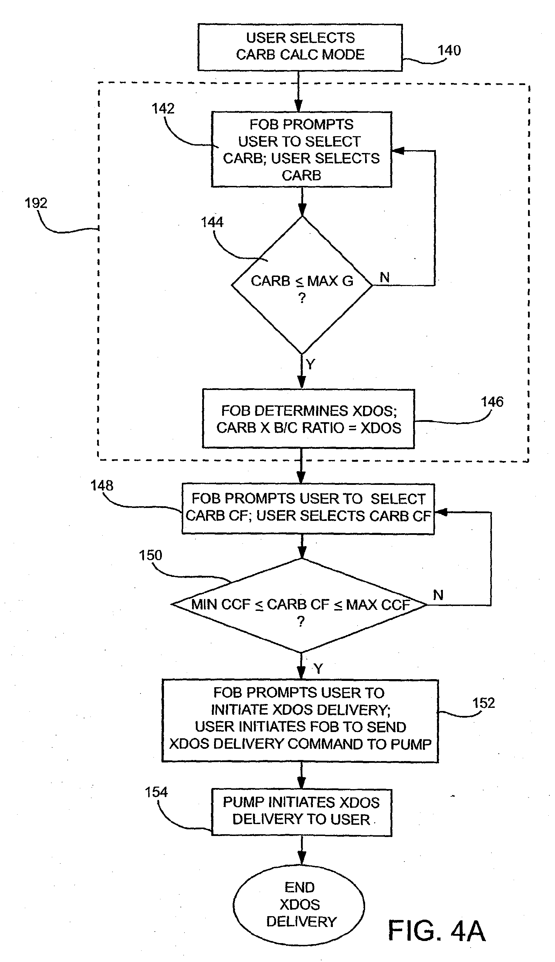

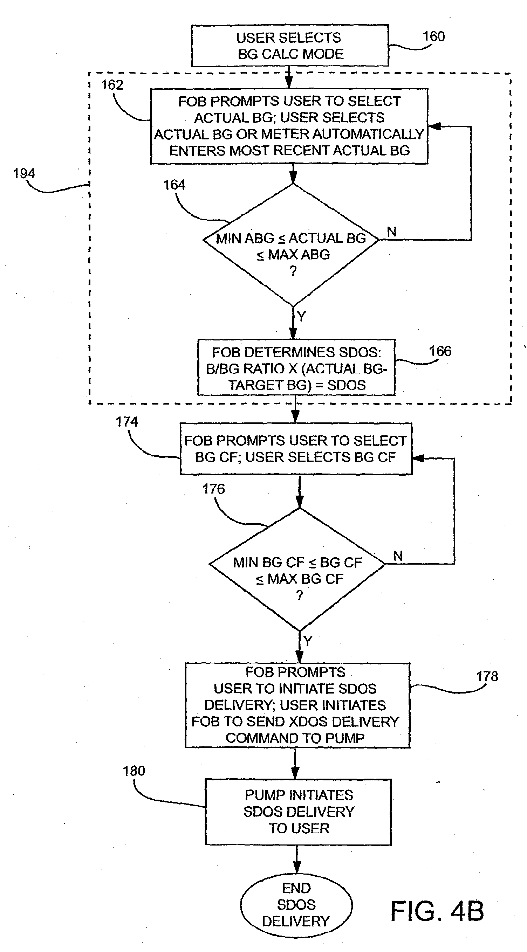

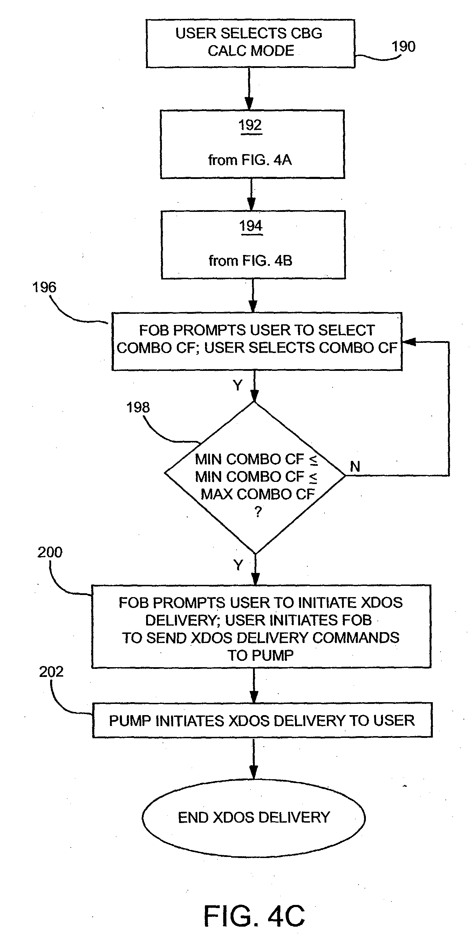

Figs. 3A, 3B and 3C respectively illustrate block diagrams of the three bolus delivery algorithms of the present invention, while Figs. 4A, 4B and 4C respectively illustrate block diagrams of the calculator function options of the present invention. In further describing the present invention, each of the three delivery algorithms will first be described in the context where no calculator function options are used, followed by a description of the three calculator functions as they apply to the standard and extended delivery algorithms. Whereas such algorithms may be implemented through the user's interface with either the pump or the fob, the following description is in the context of a user's interface with the fob, the more likely scenario.

### A Bolus Delivery Algorithms

### 1. Standard Bolus Dehverv Algorithm

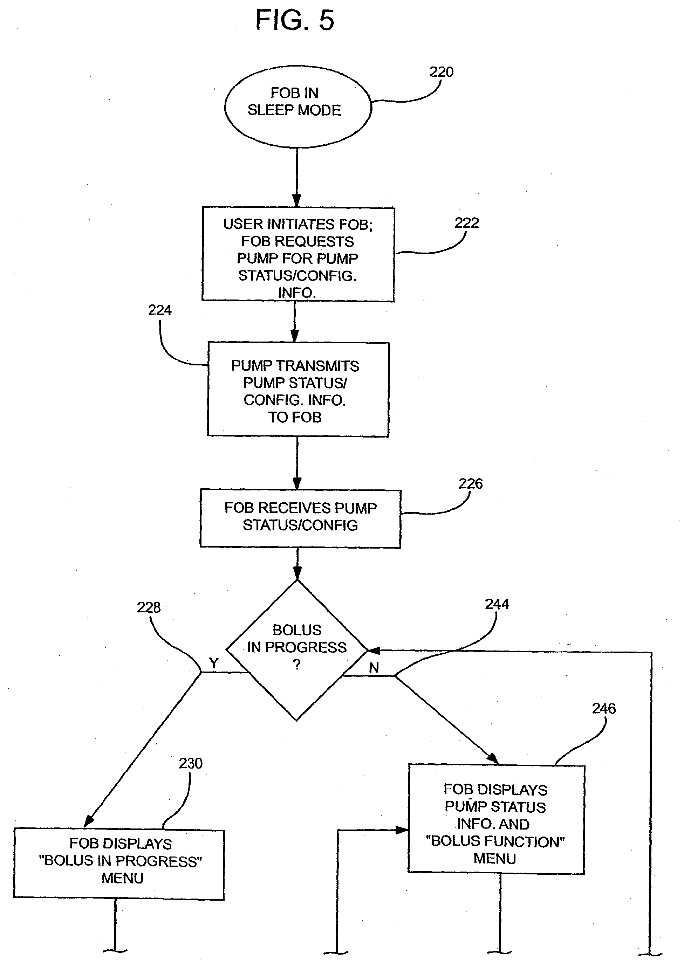

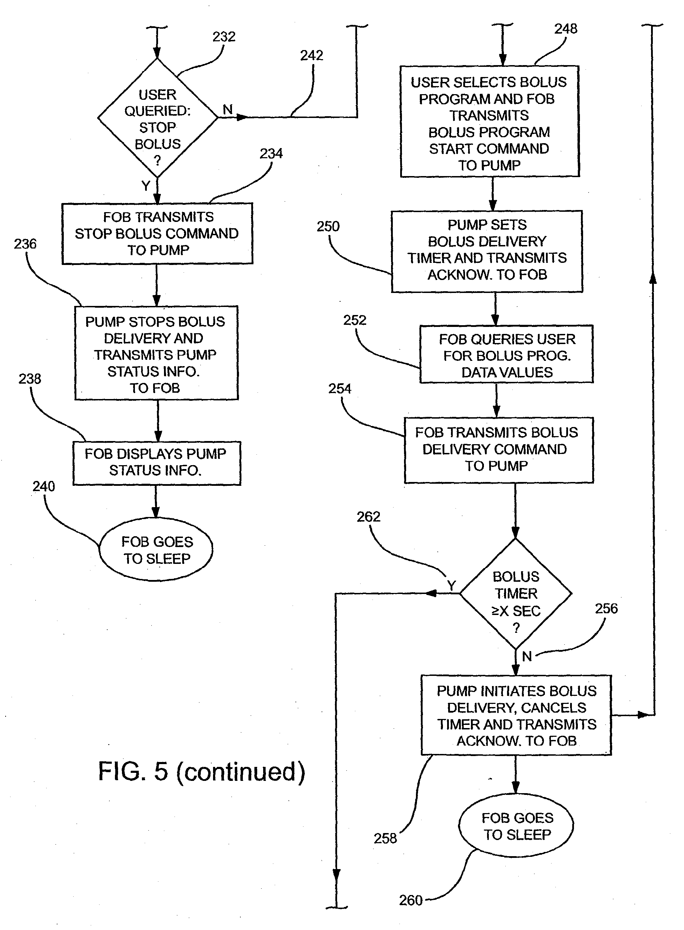

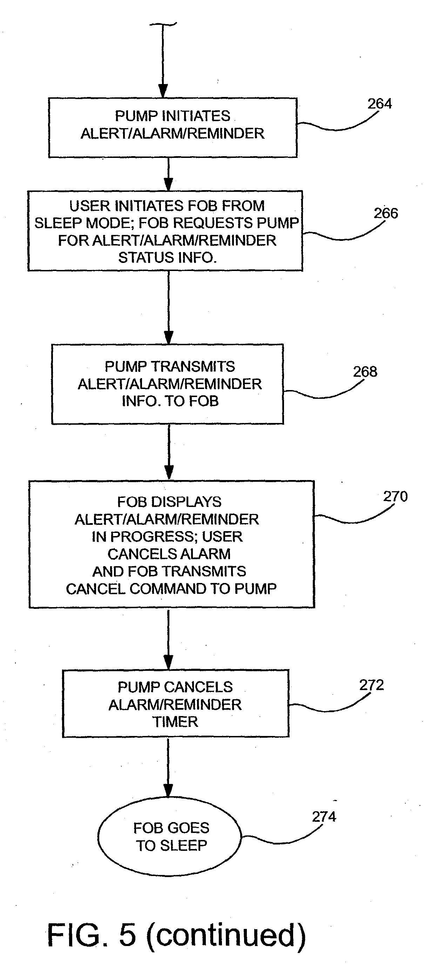

As shown in the block diagram of Fig. 3A, upon selecting the standard insulin bolus delivery program 100 from a bolus function menu (see step 246 of Fig. 5) displayed on the fob, the user is requested to set the standard bolus dosage (SDOS) 102 he or she desires to be administered. So as to prevent over-dosing, the algorithm will only implement the bolus delivery if the units entered are less than about a maximum amount (SMAX U) 104, which SMAX U will vary depending on the individual user's body mass and metabolism. For a user having an average body mass and metabolism, SMAX U will be about 10 Units. However, the SMAX U is likely to be lower for children and greater for obese users. SMAX U may be set as a default value upon the initial programming of the subject system or may be changed by the user during the programming of a particular standard bolus. Steps 102 and 104 are shown collectively referenced as 126 for purposes of describing the steps of Fig. 3C, described below. Once the bolus dosage has been set within proper limits, the user is prompted to initiate the customized bolus delivery 106. Upon initiating such SDOS delivery, the entire SDOS is immediately delivered 108 to the user.

### 2. Extended Bolus Delivery Algorithm

As shown in the block diagram of Fig. 3B, upon selecting the extended bolus delivery program 110 from a bolus function menu (see step 246 of Fig. 5) displayed on the fob, the user is requested to set the extended insulin bolus dosage (EDOS) (Units) 112 he or she desires to be delivered. So as to prevent over-dosing, the algorithm will only implement the bolus delivery if the EDOS value entered is less than a maximum amount (EMAX U) 114, which, as explained above, will vary depending on the individual user's body mass and metabolism. Next, the user is prompted to enter the desired duration of the extended bolus delivery (DTIME) 116 which time period ranges from a minimum time (MIN T) to a maximum time (MAX T). Such DTIME may range from about 1 minute to 24 hours but more typically ranges from about 30 minutes to 8 hours, for example. If the DTIME value entered is outside the acceptable range 118, the user is prompted to re-enter an acceptable value. Steps 110, 112, 114, 116 and 118 are collectively referenced as 128 for purposes of describing the steps of Fig. 3C, described below. Once these two parameters have been set within acceptable limits, the user is prompted to initiate the customized extended bolus delivery 120. Upon initiating such EDOS delivery, the EDOS is delivered to the user over DTIME 122.

### 3. Dual Bolus Delivery Algorithm

As shown in the block diagram of Fig. 3C, upon selecting the dual bolus delivery program from a bolus function menu (see step 246 of Fig. 5) displayed on the fob, the user is requested, as in the standard bolus delivery algorithm at 126 on Fig. 3A, to set the standard bolus dosage (SDOS) (Units) he or she desires to be delivered having a value that is less than a maximum amount (SMAX U), as explained above. Next, as in the extended bolus delivery algorithm at 128 of Fig. 3B, the user is requested to set the extended insulin bolus dosage (EDOS) (Units) he or she desires to be delivered, again, having a value less than a maximum (EMAX U). Additionally, the user is prompted to enter the desired duration of the extended bolus delivery (DTIME) 128 within an acceptable time range, as explained above with respect to Fig. 3B. Once these two parameters have been set within acceptable limits, the user is prompted to initiate the delivery of both the SDOS and EDOS 130. Upon initiating such SDOS and EDOS deliveries, the complete SDOS is immediately delivered 132 to the user and, upon completion of the SDOS delivery 134, the EDOS delivery is initiated 136 and continues to be delivered to the user over DTIME 138.

### B. Calculator Modes

### 1. Carbohydrate Calculator Mode

Referring now to Fig. 4A, upon selecting the carbohydrate calculator mode (CARB CALC) 140, the user is first prompted to enter the anticipated or actual grams of carbohydrates (CARB) 142 he or she intends to imminently consume. Typically, the fob is programmed to accept no more than a preselected maximum carbohydrate value (MAX G) of about 200 g, a common MAX G value for adult users having an average body mass and metabolism, but may vary depending on the body mass and metabolism of the user. If the entered CARB value is greater than MAX G 144, the user is prompted to reenter an acceptable CARB value.

Based on a preprogrammed bolus dosage to carbohydrate ratio (B/C RATIO) (Units/g), which may be changed by the user at this point, the fob controller 82 then determines the dosage of insulin (XDOS) (Units) to be delivered 146, where XDOS may be either an SDOS or an EDOS. The B/C RATIO is typically within the range from about 1 Unit:30g to 1 Unit:5g but may be more or less depending on the user's condition and needs. The value of XDOS is the product of the CARB value and the B/C RATIO value (CARB x B/C RATIO).

After the B/C RATIO is set, the user is prompted to enter a carbohydrate correction factor (CARB CF) (Units) 148 to adjust the XDOS, *i.e.,* to either decrease or increase the XDOS in order to fine-tune the bolus volume, for example, when the user anticipates exercising soon after a meal. The CARB CF value must be within a range from a minimum value (MIN CF) to a maximum value (MAX CF). For an average user, the CARB CF value typically ranges from about 0 Units to about 10 Units, but may be more or less depending on the user's needs. If the entered CARB CF value is outside an acceptable range 150, the user is prompted to reenter an acceptable CARB CF value.

Once all parameters have been set within proper limits, the fob prompts the user to initiate the XDOS delivery and, in turn, the user initiates the fob to send an XDOS delivery command 152 to the pump. The pump then initiates the XDOS delivery to the user 154.

### 2. Blood Glucose Calculator Mode

Referring now to Fig. 4B, upon selecting the blood glucose calculator mode (BG CALC) 160, the fob prompts the user to enter his or her most recent blood glucose concentration level (ACTUAL BG) (mg/dL) 162 as measured by meter 80 of fob 6 or blood glucose concentration level of most recent blood glucose test can be automatically entered by the pump controller 82 if it was generated within a defined time frame. Optionally, the ACTUAL BG may be measured by physiological fluid measurement device 300 and transmitted directly to fob 6 which allows the BG CALC to be used at a higher frequency than if meter 80 were used alone. The ACTUAL BG value must be within the range from a minimum value (MIN ABG) to a maximum value (MAX ABG). For an average user, the ACTUAL BG value typically ranges from about 0 mg/dL to 600 mg/dL, but may be more or less depending on the user's needs. If the entered value is outside this range 164, the user is prompted to reenter an acceptable ACTUAL BG value.

The fob controller 82 then determines the standard bolus dosage of insulin (SDOS) (Units) to be delivered 166 which value is the product of the bolus to blood glucose ratio (B/BG RATIO) (Units/point, where one point is equal to 1 mg/dL) and the difference between the ACTUAL BG value and the user's targeted blood glucose level (TARGET BG) (mg/dL), as defined by the following equation: B/BG RATIO x (ACTUAL BG - TARGET BG)). The B/BG RATIO is a preprogrammed value, which value may, at this point, be changed by the user within a predefined range from a minimum value. For an average user, the B/BG RATIO typically ranges from about 1 Unit:150 pt to 1 Unit:10 pt, but may be more or less depending on the user's needs. The TARGET BG is also a preprogrammed value, which value may also be changed by the user within a predefined range. For an average user, the TARGET BG may be about 60 to 250 mg/dL, but may be more or less depending on the user's needs.

Next, the fob prompts the user to enter a blood glucose correction factor (BG CF) (Units) 174 to adjust the SDOS, *i.e*., to either decrease or increase the SDOS to fine-tune the bolus volume, for example, when the user anticipates exercising soon after a meal. The BG CF value must be within a range from a minimum value (MIN BGCF) to a maximum value (MAX BGCF). For an average user, the BG CF is typically from about 0 Units to 10 Units, but may be more or less depending on the user's needs. If the entered BG CF value is outside the acceptable range 176, the user is prompted to reenter an acceptable BG CF value.

Once all parameters have been set within proper limits, the fob prompts the user to initiate the SDOS delivery and, in turn, the user initiates the fob to send an SDOS delivery command to the pump 178. The pump then initiates the SDOS delivery to the user 180.

### 3. Combined Calculator Mode

Referring now to Fig. 4C, upon selecting the combined carbohydrate/blood glucose calculator mode (CBG CALC) 190, the user is queried to enter the amount of carbohydrates he or she anticipates eating and the fob determines the XDOS based on this CARB value and the preprogrammed B/C RATIO, according to the collective steps 192 of Fig. 4A. Then, according to the collective steps 194 of Fig. 4B, the user enters his or her ACTUAL BG and the fob determines the SDOS to be delivered based on the ACTUAL BG and preprogrammed values of the user's B/BG RATIO and TARGET BG. In another embodiment of the invention, the user's ACTUAL BG is automatically transmitted to the fob which then determines the SDOS to be delivered based on the ACTUAL BG and based on preprogrammed values of the user's B/BG RATIO and TARGET BG.

The fob then prompts the user to select a correction factor (COMBO CF) (Units) 196 to adjust the XDOS if necessary, *i.e*., to either decrease or increase the XDOS to fine-tune the bolus volume, for example, when the user anticipates exercising soon after a meal. The COMBO CF value must be within a range from a minimum value (MIN COMBO CF) to a maximum value (MAX COMBO CF) 198. For an average user, the COMBO CF is typically from about 0 Units to 10 Units, but may be more or less depending on the user's needs. If the entered COMBO CF value is outside the acceptable range, the user is prompted to reenter an acceptable COMBO CF value.

Once all parameters have been set within proper limits, the fob prompts the user to initiate the XDOS delivery and, in turn, the user initiates the fob to send an XDOS delivery command to the pump 200. The pump then initiates the XDOS delivery to the user 202.

### Methods

As summarized above, the subject invention provides methods for remotely controlling medication infusion to a patient. With reference to Fig. 5, certain methods of the present invention are now described in detail. After the initial programming of the pump and fob of the subject system, typically performed by the user's physician, the fob will typically remain in a sleep mode 220 until initiated by the user 222. Upon such initiation, the fob, via the communication link, requests the pump to provide the fob with the pump status information, *e.g.,* bolus in progress status, basal in progress, remaining insulin, etc., and algorithm configuration information, *e.g*., default values including, but not limited to, B/C RATIO, B/BG RATIO, TARGET BG, bolus limits, bolus steps, programming configurations, *i.e*., extended or dual bolus functions selected, carbohydrate or blood glucose calculator mode selected. Upon receiving this request, the pump transmits the requested status and configuration information to the fob 224, which is then received by the fob 226.

If this information indicates that a bolus delivery is in progress 228, the fob will display the "Bolus in Progress" menu 230 and query the user as to whether he or she wants to stop the bolus delivery 232. If the user indicates that he or she does wish to stop the bolus delivery, the fob transmits a STOP BOLUS command to the pump 234. Upon receipt of this command, the pump stops the bolus delivery and transmits the revised pump status information to the fob 236. The fob displays this status information to the user 238, and, after a short time, goes back into sleep mode 240.

If, on the other hand, the user does not want to stop the bolus in progress 242 or no bolus is currently in progress 244, the fob displays the pump status information and the "Bolus Function" menu 246. The user then selects the desired bolus program, e.g., standard bolus program, extended bolus program or dual bolus program, and the fob transmits the bolus delivery programming start command to the pump 248. According to this command, the pump resets the bolus delivery timer and transmits an acknowledgement to the fob 250. The fob then queries the user for specific bolus program data 252, with or without the use of a calculator function, *e.g.*, carbohydrate calculator, blood glucose calculator mode or combined mode, all of which includes entering or selecting all the necessary information requested by the selected algorithms, as illustrated in Figs. 3A-3C and 4A-4C.

Upon completing the programming of the bolus delivery protocol, the user initiates the fob to transmit the bolus value and DELIVER bolus command to the pump 254. If the bolus delivery commences prior to expiration of the bolus delivery timer 256, the pump initiates the bolus delivery, cancels the timer and transmits an acknowledgment to the fob 258. After a short time, the fob returns to the sleep mode 260.

If, on the other hand, the timer expires prior to commencement of the bolus delivery 262, the pump initiates an alarm 264, either audio, motion and/or visual as described above to indicate to the user that there is a malfunction with the pump. The pump also initiates an alarm in order to alert the user to preprogrammed reminders, *e.g.*, a reminder to take a blood glucose measurement, etc. Upon being alerted by the alarm, the user initiates the fob from the sleep mode (if asleep), and the fob then requests the pump to provide the fob with the alert/alarm/reminder status 266. The pump, in turn, transmits such information to the fob 268. The fob then displays the alert/alarm/reminder currently in progress upon which the user can initiate the fob to transmit a CANCEL ALARM command to the pump 270 and take care of the cause of the alarm, *e.g*., unclog the pump infusion tubing, or perform the necessary task, take a blood glucose measurement. In response, the pump cancels the alarm and the bolus delivery timer 272. After a short time, the fob returns to the sleep mode 274.

### Kits

Also provided by the subject invention are kits for use in practicing the subject methods. The kits of one embodiment of the subject invention include at least one subject infusion pump and at least one subject fob, as described above. The kits may also include one or more pump infusion sets and/or one or more test strips compatible for use with the fob's meter. In another embodiment of the subject invention, the kits include at least one subject measurement device and at least one fob. Other kits include at least one infusion pump, at least one measurement device and at least one fob. The kits may further include software programs recorded on a CD-ROM or the like, which programs may be downloaded to the pump and/or fob by the user or physician by means of an external device, such as a computer. Finally, the kits may further include instructions for using the subject devices. These instructions may be present on one or more of the packaging, label inserts or containers within the kits, or may be provided on a CD-ROM or the like.

It is evident from the above description and discussion that the above-described invention provides a simple, convenient and discrete way of administering a medication protocol to a patient. The present invention minimizes the number of devices that a patient must carry with him or her in order to effectively administer medication and monitor its effects on the patient. The present invention also maximizes the flexibility and real-time control that a patient has over administration of his or her medication. As such, the subject invention represents a significant contribution to the art.

All publications and patents cited in this specification are herein incorporated by reference as if each individual publication or patent were specifically and individually indicated to be incorporated by reference. The citation of any publication is for its disclosure prior to the filing date and should not be construed as an admission that the present invention is not entitled to antedate such publication by virtue of prior invention.

Although the foregoing invention has been described in some detail by way of illustration and example for purposes of clarity of understanding, it is readily apparent to those of ordinary skill in the art in light of the teachings of this invention that certain changes and modifications may be made thereto without departing from the spirit or scope of the appended claims.

## Claims

1. A system comprising:
a medication infusion pump configured to be worn on the body of a patient;
a physiological fluid monitoring device configured to be worn on the body of a patient for substantially continuous monitoring of at least one characteristic of physiological fluid; and
a remote control device for remotely controlling the medication infusion pump and the physiological fluid monitoring device, wherein the remote control device comprises a physiological fluid monitoring means for the episodic monitoring of at least one characteristic of physiological fluid.

2. The system of claim 1 further comprising means for communicating between the remote control device, the medication infusion pump and the physiological fluid monitoring means.

3. The system of claim 1 or 2 wherein said remote control device comprises:
a communication module for communicating with the medication infusion pump and with the physiological fluid monitoring device;
one or more control keys for user interface with the remote control device; and
a controller for controlling the transfer and receipt of data to and from the communication module and for processing user interface data

4. The system of any of claims 1 to 3 wherein said remote control device further comprises a port for operatively receiving physiological fluid test strip.

5. The system of any of claims 1 to 4 wherein said medication infusion pump further comprises:
a communication module for communicating with the remote control device;
one or more control keys for user interface with the medication infusion pump; and
a controller for controlling the transfer and receipt of data to and from the remote control device and for processing user interface data.

6. The system of any of claims 1 to 5 wherein said physiological fluid monitoring device comprises:
a physiological fluid sampling means; and
a sensor for measuring the concentration of one or more analytes within physiological fluid, wherein said sensor is operatively connected and in fluid communication with said physiological fluid sampling means.

7. The system of claim 6 wherein said physiological fluid monitoring device further comprises:
a communication module for communicating with the remote control device;
one or more control keys for user interface with the medication infusion pump; and
a controller for controlling the transfer and receipt of data to and from the remote control device and for processing user interface data.

8. A system for administration of medication, comprising:
a medication infusion pump configured to be worn on the body of a patient;
a remote control device for remotely controlling the medication infusion pump; and
a processor associated with said remote control device; and
software for use with said processor for implementing medication delivery protocols by said medication infusion pump, said medication delivery protocols comprising a first medication delivery protocol for the immediate infusion of a selected dosage of medication, a second medication delivery protocol for the infusion of a selected dosage of medication over a selected period of time and a third medication delivery protocol for the immediate infusion of a first selected dosage of medication followed by the infusion of a second selected dosage of medication over a selected period of time.

9. The system of 8 wherein said software comprises algorithms for calculating a patient's current or substantially current blood glucose level or the patient's anticipated or actual carbohydrate intake and for modifying said medication delivery protocols according to said blood glucose level or said carbohydrate intake.

10. A method of monitoring and controlling the concentration of a physiological fluid analyte of patient, comprising:
episodically measuring the concentration of the analyte from a sample of physiological fluid taken from the patient, wherein the episodic measuring is performed using a remote device;
substantially continuously sampling the physiological fluid of the patient using percutaneous means;
substantially continuously measuring the concentration of the analyte within the sampled physiological fluid;
communicating data representative of the analyte concentration to the remote device;
determining whether the analyte concentration falls outside an acceptable range; and
adjusting a medication delivery protocol upon a determination that the analyte concentration falls outside the acceptable range.

11. A method of monitoring and controlling a patient's glucose level, comprising:
episodically measuring the concentration of glucose from a sample of physiological fluid taken from the patient;
providing a value representative of the patient's carbohydrate intake;
calculating a dosage of insulin to be administered to the patient based on said glucose concentration and based on said carbohydrate intake value;
transmitting a radio frequency signal representative of said dosage to an insulin infusion pump worn by the patient, said infusion pump comprising a radio frequency receiver for receiving said radio frequency signal; and
administering said dosage of insulin to the patient by means of said infusion pump.
